# FASCICULE DE BREVET EUROPEEN

(11) **EP 4 335 384 B1**
(45) Date de publication et mention de la délivrance du brevet: **04.06.2025**
(21) Numéro de dépôt: 22194759.1
(22) Date de dépôt: 09.09.2022
(51) Int. Cl.: A61B 10/00

(54) **DISPOSITIF POUR TRAITER UN ÉCHANTILLON DE MATIÈRE FÉCALE**
VORRICHTUNG ZUR BEHANDLUNG EINER FÄKALEN MATERIALPROBE
DEVICE FOR TREATING A FECAL SAMPLE

(43) Date de publication de la demande: 13.03.2024
(73) Titulaire: Bio-X Diagnostics, 5580 Rochefort (BE)
(72) Inventeur: LEJEUNE, Johan, 56130 Nivillac (FR); VERSMISSE, Yann, 22120 Pommeret (FR)
(74) Mandataire: Gevers Patents

(56) Documents cités:
- CN-A- 110 806 332
- CN-U- 204 008 178
- CN-U- 206 208 562
- CN-U- 210 571 496
- US-A1- 2019 134 541

## Description

### Domaine technique

L'invention divulguée dans ce document concerne des dispositifs pour traiter des échantillons biologiques aux fins de diagnostics médicaux, notamment dans le domaine de la médecine vétérinaire. Dans le cas présent, ces échantillons sont des échantillons de matière fécale animale (ou fèces ou prélèvement d'environnement contenant de la matière fécale), par exemple de ruminants.

### Art antérieur

Dans le cadre du domaine technique susmentionné, il est connu de traiter un échantillon de matière fécale d'un animal en vue d'analyses et/ou de tests, par exemple afin de détecter des micro-organismes et/ou des maladies de cet animal. Un tel « traitement » comprend généralement les étapes suivantes :
- placer l'échantillon de matière fécale dans un flacon contenant une solution de traitement, typiquement de mise en suspension de l'échantillon,
- mélanger la solution et l'échantillon dans le flacon, typiquement par agitation du type vortex,
- prélever du surnageant du mélange ainsi obtenu.
Les tests et/ou analyses nécessaires sont ensuite effectués sur le surnageant de manière connue de l'homme du métier.

Lors de ce traitement, il est souhaitable de limiter les manipulations tant pour des raisons d'efficacité que pour ne pas contaminer l'échantillon. Il est également nécessaire de calibrer précisément la quantité de matière fécale dans l'échantillon pour que les tests et/ou analyses soient reproductibles.

Effectuer ce traitement de façon approximative au moyen d'un simple flacon et d'une spatule n'est donc pas souhaitable. Afin de fournir un dispositif adéquat au traitement d'un tel échantillon, la publication de brevet EP3185782B1 divulgue un flacon muni d'un bouchon supérieur à la surface interne duquel est connecté le manche d'une cuillère s'étendant dans le flacon. Le manche prend la forme d'une tige, et la cuillère comprend une partie creuse calibrée pour prélever l'échantillon. Le bouchon est pourvu d'une ouverture de sortie et d'un filtre agencé pour filtrer le mélange de l'échantillon avec une solution de traitement lors de son passage de l'intérieur vers l'extérieur du flacon via l'ouverture.

Avec le dispositif du document EP3185782B1, il est ainsi rendu possible de prélever l'échantillon avec la cuillère, de placer la cuillère avec l'échantillon dans le flacon, de mélanger l'échantillon avec une solution de traitement présente dans le flacon, et de prélever du mélange ainsi obtenu en retournant le flacon pour que le mélange passe successivement par le filtre et l'ouverture du bouchon.

Ce dispositif, bien que facilitant le traitement de l'échantillon, est susceptible d'être défectueux lors du prélèvement du surnageant par retournement vu qu'une sédimentation du mélange peut se produire dans le flacon et obstruer le filtre. Ce défaut est marqué pour une matière fécale particulièrement hétérogène comme celle d'un ruminant.

Des dispositifs apparentés pour lesquels un moyen de filtrage est agencé au niveau d'un manche de cuillère sont en outre divulgués dans les publications US 2019/134541 A1, sur laquelle est basé le préambule de la revendication 1, CN 110 806 332 A, CN 204 008 178 U, CN 210 571 496 U, et CN 206 208 562 U.

### Exposé de l'invention

Un objet de la présente invention est de fournir un dispositif pour traiter un échantillon de matière fécale plus efficace et pratique à utiliser. En particulier, il est souhaité de fournir un tel dispositif qui soit efficace et pratique à utiliser, et qui permette de prélever une quantité précisément calibrée de matière fécale tout en limitant les manipulations nécessaires à son traitement.

L'invention propose, dans ce but, un dispositif pour traiter un échantillon de matière fécale selon la revendication 1.

Le dispositif selon la présente invention reproduit certaines caractéristiques du document EP3185782B1, notamment le flacon comprenant le contenant et le bouchon à partir duquel s'étend le manche de la cuillère, les avantages liés à ces caractéristiques étant dès lors également reproduits par ce dispositif. Toutefois, le dispositif selon l'invention se distingue de celui du document EP3185782B1 par la modification en position du moyen de filtration (i.e. les orifices de filtration) et en structure du manche qui induisent en outre un moyen de sortie distinct pour le mélange contenu dans le contenant. En effet, le manche de la cuillère est creux, ou, du moins, il comprend une cavité interne qui est en communication fluidique avec l'embout et avec l'intérieur du contenant via les orifices de filtration. Ainsi, le prélèvement du surnageant du mélange peut se faire en retournant le contenant de sorte que le mélange passe par les orifices de filtration du manche, puis dans la cavité interne, jusqu'à l'embout du bouchon. Comme les orifices de filtration ne sont pas au niveau du bouchon, mais dans le manche de la cuillère, davantage à l'intérieur du contenant, ils ne sont pas obturés par de la matière fécale sédimenté lors du retournement du contenant. Le prélèvement du surnageant par ce moyen de sortie peut ainsi se faire de façon pratique et efficace.

La paroi consiste en une paroi arrière du manche prolongeant le fond de la partie opérante. Les orifices de filtration sont de la sorte présents sur la paroi arrière du manche, soit la paroi qui prolonge le fond de la partie opérante de la cuillère, ou en termes plus familiers, le dos de la cuillère. Ils sont sur le côté du manche opposé à la surface creuse de la partie opérante de la cuillère. Cette position avantageuse protège les orifices de filtration d'une éventuelle obstruction par de la matière fécale lors de l'étape de prélèvement de l'échantillon.

Les orifices de filtration ne sont au contraire pas présents sur une paroi avant du manche bordant la cavité interne et opposée à la paroi arrière, c'est-à-dire la paroi à partir de laquelle est formée la surface creuse de la partie opérante, ou en termes plus familiers, l'intérieur ou le creux de la cuillère. Cette paroi est pleine, et donc sans orifice. De cette façon, il est possible de racler de façon continue de la matière fécale sur la paroi avant et sur le dessus de la partie opérante de la cuillère pour ajuster la quantité de matière fécale à introduire dans le contenant. Cette opération de raclage peut être réalisée sur un bord d'ouverture du contenant fermable par le bouchon. La matière fécale de l'échantillon est ainsi très précisément calibrée. Comme la paroi avant est sans orifice de filtration, d'une part ceux-ci ne sont pas bouchés lors de ce raclage, et d'autre part, aucun élément de matière fécale indésirable n'est introduit par force dans la cavité interne. De plus, ce raclage est rendu possible de façon pratique, d'une seule main, sans autre instrument que la cuillère, ce qui réduit le nombre de manipulations et les risques de contamination de l'échantillon (pas de spatule, ...), et accroit la reproductibilité ainsi que la quantification du traitement par la calibration particulièrement précise de l'échantillon.

Ces modes de réalisation n'excluent bien sûr pas la présence d'orifices de filtration sur d'autres parties du manche sauf sur la paroi avant dans le cadre de l'invention, par exemple sur une paroi latérale du manche reliant les parois avant et arrière, même si, plus préférentiellement, ces orifices ne sont présents que sur la paroi arrière, pour les raisons mentionnées ci-dessus.

Lesdites parois avant et arrière sont planes et parallèles. s'agit d'une réalisation particulière du terme « parois opposées » pour laquelle les effets techniques susmentionnés de non obstruction de la paroi arrière et d'éventuel raclage continu du dessus de la partie opérante via et au niveau de la paroi avant sont exacerbés. La réalisation de parois planes est en outre aisée, par exemple, dans le cas préféré d'un manche de forme prismique, par exemple parallélépipédique. Dans ce cas, la paroi arrière prolonge de préférence le fond de la partie opérante selon un même plan, ce qui rend plus aisée la constitution de la cuillère et optimise la protection des orifices de filtration contre l'obturation par de la matière fécale.

Plus généralement, le manche est de préférence d'une forme prismique, qui est préférentiellement associée à un prisme droit, et qui peut être potentiellement déformée dans le sens où chaque face du prisme peut comprendre des points en lesquels sa courbure est non nulle, sauf les parois avant et arrière.

Dans ce cas, une face du prisme correspond en la paroi avant et/ou une autre face du prisme opposée du prisme correspond en la paroi arrière pour les modes de réalisation faisant intervenir une et/ou l'autre de ces parois.

Dans le cadre de ce document, le terme « paroi », en référence à une partie du manche, désigne typiquement une partie latérale et/ou extrémale et/ou de bord du manche séparant l'intérieur (par exemple, la cavité interne ou bien une partie interne pleine du manche) et l'extérieur du manche (en l'occurrence l'intérieur du contenant lorsque le bouchon ferme celui-ci). Une telle paroi a généralement une épaisseur de moins de 3 mm, et de préférence comprise entre 0,5 et 2,5 mm, par exemple 1,6 mm.

Dans le cadre de ce document, le terme « orifice de filtration » est utilisé afin de désigner des orifices dans la paroi (arrière) du manche ayant une fonction de filtration. En d'autres termes, ces orifices permettent uniquement aux particules du mélange hétérogène formé par l'échantillon et la solution de traitement d'une taille suffisamment petite de pénétrer dans la cavité interne, ces particules formant ou à tout du moins comprenant le surnageant à prélever. Ceci est avantageux dans le cas où la matière fécale est celle d'un ruminant car elle comprend alors beaucoup de fibres et d'éléments de grande taille (de la paille, etc.) qu'il n'est pas souhaité de prélever aux fins des tests et/ou analyses ultérieurs. Cette filtration du mélange se fait en particulier de part et d'autre de la paroi (arrière), à partir de l'extérieur du manche vers l'intérieur du manche. En particulier, les orifices de filtration induisent une communication fluidique de part et d'autre la paroi (arrière), ce qui permet au surnageant d'atteindre l'embout du bouchon à partir de l'intérieur du contenant en transitant avantageusement par le manche de la cuillère.

Tout ou partie des orifices de filtration définissent de préférence chacun une forme tronconique dans la paroi (arrière) orientée vers la cavité interne, donc de diamètre de section décroissant du contenant vers la cavité interne. Ce diamètre de section est de préférence compris entre 0,10 et 1,00 mm. En d'autres termes, le diamètre d'un orifice de filtration au niveau intérieur de la paroi est alors plus petit que le diamètre de l'orifice de filtration au niveau extérieur de la paroi. Ces diamètres sont de façon préférée et respective compris entre 0,20 mm et 0,50 mm, par exemple 0,35 mm, et entre 0,60 et 1,00 mm, par exemple 0,80 mm.

Avantageusement, les orifices de filtrations selon cette réalisation sont très simples à réaliser sous la forme de perforations et ne nécessite pas d'utilisation de filtre particulier ou complexe permettant une filtration très fine de l'ordre d'une ou deux dizaines de µm ou moins. Ces orifices sont notamment suffisants dans le but d'éviter une pénétration dans la cavité interne de fibres et/ou de particules de grande taille de matière fécale.

L'usage, dans ce document, du verbe « comprendre », de ses variantes, ou de leurs conjugaisons, ne peut en aucune façon exclure la présence d'éléments autres que ceux mentionnés. De la même façon, l'usage, dans ce document, de l'article indéfini « un », « une », ou de l'article défini « le », « la » ou « l' », pour introduire un élément n'exclut pas la présence d'une pluralité de ces éléments.

Dans le cadre de la présente invention, le contenant est de préférence muni d'une ouverture supérieure, c'est-à-dire à l'opposé d'une surface de fond (ou base) adaptée pour reposer sur un support lorsque le contenant contient la solution de traitement de façon à maintenir celle-ci dans le contenant sans l'aide du bouchon malgré la gravité. L'ouverture supérieure est de préférence la seule ouverture du contenant. Le bouchon ferme le contenant, typiquement de façon hermétique et/ou étanche, par exemple, autour d'une telle ouverture supérieure, et par exemple par vissage.

Le manche est typiquement solidarisé au bouchon via une fixation et/ou un couplage mécanique sur une surface interne du bouchon, c'est-à-dire une partie du bouchon dirigée vers l'intérieur du contenant, vu que la cuillère s'étend dans le contenant lorsque celui-ci est fermé par le bouchon. Le manche de la cuillère peut être solidarisé avec le bouchon de différentes façons dans le cadre de l'invention. Le manche peut ainsi être fixé directement ou via un élément intermédiaire au bouchon de façon permanente. Le manche peut alternativement être solidarisé de façon amovible au bouchon, par exemple par un couplage mécanique amovible. Par exemple, un mouvement relatif en rotation entre le bouchon et la cuillère peut être possible. Le caractère amovible de la solidarisation présente l'avantage de pouvoir détacher la cuillère du bouchon pour nettoyer plus aisément la cuillère, le bouchon et/ou le contenant, et/ou remplacer plus spécifiquement la cuillère ou le bouchon si une seule de ces pièces est endommagée.

De préférence, la cuillère comprend un rebord s'étendant à une extrémité du manche opposée à la partie opérante, et par exemple de façon perpendiculaire au manche, ce rebord comprenant des renforts coopérant (de façon amovible) avec des languettes de maintien agencés au niveau d'une surface interne du bouchon. Ce mode de réalisation particulièrement simple permet un maintien de la cuillère résistant et fiable au niveau du bouchon, ce qui permet d'effectuer le mélange de la solution de traitement et de l'échantillon par agitation, par exemple mécanique, sans risquer que la cuillère se détache en partie ou en totalité du bouchon lors de cette opération.

Selon un mode de réalisation de l'invention, le contenant s'étend autour d'un axe et, lorsque le bouchon ferme le contenant, au moins 15%, préférentiellement au moins 20%, par exemple, environ 25%, en volume du contenant s'étend au-dessus des orifices de filtration le long de l'axe, et/ou, de préférence « et », au moins 15%, de préférence au moins 20%, par exemple, environ 25%, en volume du contenant s'étend en-dessous des orifices de filtration le long de l'axe.

Le contenant comprend ainsi un espace sous les orifices de filtration et un espace au-dessus de ceux-ci qui utilisables comme des zones de sédimentation pour les particules de grosse taille (des fibres, de la paille, etc.) du mélange tant lorsque le contenant repose sur une base, par exemple dans une configuration de repos, que lorsqu'il est retourné, par exemple afin de délivrer du surnageant. Les orifices de filtration sont ainsi agencés « vers le milieu », plus préférentiellement, dans la moitié centrale considérée selon l'axe, du contenant, dans une zone sans intersection avec les zones de sédimentation ou suffisamment éloignée de celles-ci. En particulier, ces zones de sédimentation sont suffisantes pour contenir toutes les particules de grosse taille, et les orifices de filtration ne sont alors pas obturés par de la matière fécale lors du traitement de l'échantillon, ce qui garantit une meilleure reproductibilité, possible quantification, et fiabilité de ce dernier.

De préférence, le contenant est de forme tubulaire d'une hauteur mesurée le long de l'axe au moins trois fois plus grande, par exemple quatre fois plus grande, qu'un diamètre de section transverse du contenant mesuré perpendiculairement à l'axe. Cette forme tubulaire allongée selon l'axe est particulièrement adaptée à la sédimentation et permet d'agencer les orifices de filtration le long de l'axe sur une grande distance lorsque le bouchon ferme le contenant. En particulier, la cuillère est préférentiellement dimensionnée pour s'étendre dans le contenant, à partir du bouchon, le long de l'axe, sur au moins 70%, par exemple 80%, de cette hauteur. Selon un exemple numérique non limitatif, et représenté dans les figures ci-après introduites, la hauteur est de 11,00 cm, le diamètre de section transverse est de 2,75 cm, la cuillère s'étend le long de l'axe sur 8,50 cm à partir du bouchon, et les orifices de filtration sont présents sur le manche de la cuillère entre 2,5 cm et 5,5 cm calculés le long de l'axe à partir du bouchon. Dans un tel dispositif, il a été déterminé que les zones de sédimentation devraient s'étendre sur au moins 2 cm le long de l'axe. Le diamètre de section transverse susdit, ainsi que toute variation de moins de 10% de celui-ci, sont avantageusement adaptés à être placés sur un portoir standard de laboratoire, et notamment sur une plateforme d'agitation pour mélanger l'échantillon et la solution de traitement.

Le contenant selon ces modes de réalisation préférés est avantageusement d'une grande capacité et permet donc d'utiliser une cuillère dont la partie opérante peut également contenir une grande quantité de matière fécale. Ceci présente un avantage par rapport à l'état de la technique pour lequel des cuillères de petite capacité sont utilisées. En effet, une petite cuillère induit potentiellement un besoin de répétition de l'opération de prélèvement de matière fécale, donc davantage de manipulation et une plus grande incertitude sur la quantité de matière fécale qui est réellement prélevée (et donc une calibration moins précise). Par exemple, la partie opérante de la cuillère permet de prélever environ (c'est-à-dire, à 10% près) 4,5 g de matière fécale (quantifiée à l'état humide). De cette façon, non seulement une seule opération de prélèvement est nécessaire avec cette cuillère, mais en outre, la quantité de matière fécale peut être calibrée précisément comme il est exposé précédemment.

Le contenant de forme tubulaire, ou de révolution autour de l'axe, comprend de préférence une base inférieure jupée sur laquelle il peut reposer. Ceci permet au contenant de tenir sans portoir, par exemple, sur une paillasse ou au sein d'un poste de sécurité microbiologique.

Suivant un mode de réalisation de l'invention, le contenant est constitué de polyéthylène basse densité linéaire. Par exemple, il est constitué de Flexirene ^{®}. Ce matériau constitue un très bon compromis entre le besoin d'avoir un contenant assez souple pour pouvoir être pressé afin d'activer et/ou de faciliter la distribution du mélange par l'embout, et celui d'avoir un contenant suffisamment rigide pour garantir son étanchéité, éviter d'éventuelles déformations et garder une régularité de production.

Selon un mode de réalisation de l'invention, le fond et le bord latéral de la partie opérante définissent un espace creux de forme parallélépipédique rectangle de coins et/ou angles de préférence arrondis. Cet espace creux définit au final ce qui est familièrement appelé le « creux de la cuillère », soit l'endroit au sein duquel la matière fécale prélevée avec la cuillère est principalement placée.

La forme de nature rectangulaire n'est pas choisie au hasard et se distingue avantageusement des formes de tels espaces creux connues de l'art antérieur qui sont en général davantage « rondes », par exemple, sphérique, ellipsoïdique, ou de formes apparentées. En effet, cette forme plus « angulaire » permet d'éviter la formation d'un effet vortex au niveau de l'espace creux lors d'une étape ultérieure de mélange de l'échantillon avec une solution de traitement. En effet, cette étape est typiquement effectuée par agitation mécanique régulière, par exemple du type vortex, ce qui peut induire un effet vortex au sein même de l'espace creux qui ne permet alors pas une bonne extraction de l'échantillon de l'espace creux. Or, dans le cas où l'échantillon ne se détache pas bien de la cuillère, son mélange avec la solution de traitement ne permet pas une homogénéisation nécessaire aux étapes ultérieures du traitement de l'échantillon et/ou aux tests et/ou analyses ultérieurs. Une forme « ronde » ou de régularité semblable pour l'espace creux permet plus aisément la formation non souhaitée d'un tel effet vortex. L'utilisation d'une forme de section rectangulaire, ou plus généralement polyédrique, et de façon préférée parallélépipédique rectangle, est avantageuse car elle crée des turbulences lors de l'étape de mélange, et ces turbulences contribuent au détachement aisé de la matière fécale de la partie opérante de la cuillère.

Il s'agit également d'un avantage significatif par rapport à l'art antérieur de la publication de brevet EP3185782B1 cité en début de document. En effet, dans ce document, l'espace creux de la partie opérante de la cuillère est de section ayant une forme oblongue, ce qui induit l'utilisation d'un moyen de mise en suspension mécanique additionnel dans le contenant pour réaliser de façon appropriée le mélange pour les raisons exposées ci-dessus. Grâce à la forme de l'espace creux pour le dispositif selon l'invention, il n'est pas nécessaire d'utiliser un tel moyen de mise en suspension mécanique, ce qui rend le dispositif plus aisé à fabriquer et à utiliser.

La forme parallélépipédique rectangle est de préférence de section parallèle au fond de la partie opérante d'une longueur au moins 50%, et de préférence au moins deux fois, plus grande qu'une largeur. La longueur est mesurée en général selon l'axe autour duquel s'étend le contenant et le long duquel s'étend la cuillère. La largeur est mesurée perpendiculairement à l'axe et parallèlement au fond. La longueur est par exemple d'environ 3,5 cm et la largue est par exemple d'environ 1,5 cm.

Comme le terme « forme » le suppose, la forme parallélépipédique rectangle d'espace creux n'impose pas à strictement parler que ledit espace creux soit un parallélépipède rectangle, mais plutôt ait une forme qui s'en rapproche fortement. Ainsi, les coins et/ou angles de cette forme sont de préférence (un peu) arrondis, sans bien sûr mener à une forme de section oblongue (car auquel cas, la forme ne serait plus celle d'un parallélépipède). Ceci permet de faciliter le détachement de l'échantillon de la partie opérante lors de son mélange avec la solution de traitement. En effet, tel que le comprendra l'homme du métier, les angles et/ou les coins sont des zones au sein desquels il n'est pas aisé d'extraire la matière fécale lors de cette étape.

Le manche prolonge préférentiellement continument la partie opérante selon une même (et seule) forme prismique, de préférence relative à un prisme droit. De préférence, la partie opérante (ou plus précisément l'espace creux susmentionné) est alors formée dans cette forme. Le manche n'est donc notamment pas « plus fin » dans ce mode de réalisation que la partie opérante de la cuillère. La cuillère selon ce mode de réalisation est ainsi conçue continument d'une seule pièce de forme prismique, ce qui la rend particulièrement résistante lors de son usage, et facilite également sa fabrication à partir d'un même corps de matériau.

Dans une réalisation plus préférée, le prime droit est un parallélépipède de base rectangulaire, cette forme étant particulièrement aisée à fabriquer et à tenir en main, ce qui facilite donc l'utilisation de la cuillère et, au final, du dispositif. La forme prismique a de préférence des (ses) coins et/ou angles (externes) (un peu) arrondis, ce qui facilite l'élimination de matière fécale excédentaire à l'échantillon lors de son prélèvement, et donc facilite l'utilisation de la cuillère.

Il est ci-après introduit des modes de réalisation préférés et avantageux du bouchon du dispositif. Ces modes de réalisation facilitent l'utilisation du bouchon, donc du dispositif, d'une seule main, en évitant en outre l'utilisation d'instruments additionnels de prélèvement du mélange tels qu'une seringue, et contribuent donc synergiquement avec la cuillère à rendre le dispositif à la fois plus pratique et plus efficace à utiliser. En particulier, ladite utilisation d'une seule main suit du fait que les moyens d'ouverture du bouchon selon ces modes de réalisation sont solidaires du bouchon et qu'il n'est dès lors pas nécessaire d'utiliser une main pour tenir le contenant et une autre main pour tenir le bouchon et le déposer à côté du contenant comme dans le document EP3185782B1.

Selon un tel mode générique de réalisation préféré du bouchon du dispositif, le bouchon comprend :
- une partie inférieure munie :
   - d'éléments de fixation amovible au contenant, autour d'une ouverture supérieure de celui-ci, et
   - d'au moins une ouverture permettant une communication fluidique entre l'embout du bouchon et un espace intérieur de la partie inférieure du bouchon prolongeant continument la cavité intérieure ;
- une partie supérieure surmontant la partie inférieure et couplée de façon mécanique à celle-ci, de sorte qu'elle puisse être déplacée par rapport à celle-ci entre :
   - une configuration de fermeture empêchant une distribution du mélange par l'embout, et
   - une configuration d'ouverture permettant cette distribution.
Le bouchon selon ce mode de réalisation comprend donc une structure double ou de double-bouchon.

Avantageusement, cette structure double constituée des parties inférieure et supérieure permet une implémentation simple et pratique du bouchon. Elle permet d'une part de fermer et/ou de moduler l'ouverture du contenant et de connecter de façon fluidique et continu la cavité interne du manche et l'embout, et d'autre part, de supporter le couplage mécanique et les moyens permettant une commutation aisée du bouchon entre les configurations de fermeture et d'ouverture. Comme il est expliqué ci-avant, le contenant peut être de forme tubulaire et standardisé, la partie inférieure pouvant être dimensionnée de façon à s'adapter hermétiquement à l'ouverture supérieure du contenant. Cette dernière surmonte de préférence le contenant, et correspond de préférence à la seule ouverture du contenant.

Typiquement, l'actionnement (resp. la désactivation) des éléments de fixation amovible pour fixer (resp. retirer) la partie inférieure au (resp. du) contenant correspondent à la fermeture (resp. l'ouverture) du contenant par le bouchon lorsque la partie supérieure est dans la configuration de fermeture.

Les éléments de fixation amovible incluent de préférence un filetage interne, c'est-à-dire un filetage sur une surface interne bordant l'espace intérieur, agencé pour coopérer, typiquement hermétiquement et de façon étanche, avec un filetage externe du contenant, autour de son ouverture. Dans le cadre de ce document, le terme « filetage » (ou filet) couvre une structure hélicoïdale en relief par rapport à un support, le relief pouvant soit être creusé dans le support, soit être protubérant, tel que connu de l'homme du métier. De préférence, le pas de vis est optimisé de pour constituer un compromis entre un besoin d'étanchéité de la fixation amovible et un besoin rendre cette fixation rapide et pratique. Par exemple, le diamètre des hélicoïdes correspondantes est d'environ (à 10% près) 2,75 cm, et le nombre de tour est d'environ 2,0. Le nombre de tours à effectuer pour fermer le contenant est ainsi petit, de sorte que cette opération peut être effectuée d'une main en évitant des troubles musculosquelettiques. De préférence, les hélicoïdes correspondant à ces filetages sont inclinées, pour renforcer le caractère étanche de la fixation par vissage.

De préférence, la partie inférieure comprend une partie striée externe pour faciliter la préhension de celle-ci par une seule main, et rendre le dévissage et le vissage de la partie inférieure sur le contenant pratique et plus aisée.

La partie inférieure du bouchon comprend de préférence également une pointe surmontant la au moins une ouverture. La pointe permet de guider la distribution du mélange et d'empêcher la formation de goutte lors du passage de la partie supérieure du bouchon de la configuration d'ouverture à la configuration de fermeture. La « au moins une ouverture » de la partie inférieure comprend de préférence une pluralité d'ouvertures entourant la pointe pour délivrer le mélange de façon progressive et contrôler. Chaque telle ouverture est de petite dimension, de préférence, de diamètre maximal d'extension compris entre 0,5 et 3,0 mm, par exemple 1,0 mm ou 1,5 mm.

La partie supérieure du bouchon comprend préférentiellement une portion supérieure dimensionnée pour envelopper au moins partiellement, de préférence totalement, cette pointe et percée par un trou débouchant supérieur dimensionné pour accueillir la pointe de sorte qu'il devienne hermétiquement obturé lorsque la partie supérieure est dans la configuration de fermeture. Cette partie supérieure peut avantageusement être manipulée de façon aisée et pratique, la commutation entre les configurations de la partie supérieure tenant à la position d'obturation ou de non obturation du trou par la pointe.

La portion supérieure de la partie supérieure présente également l'avantage d'être *a fortiori* allongée vu qu'elle peut envelopper la pointe, cette forme allongée facilitant la distribution du mélange dans des tubes de test de petits formats.

Le couplage mécanique entre la partie supérieure et la partie inférieure du bouchon est de préférence réalisée par la coopération, typiquement hermétique et étanche, de deux filetages respectivement au niveau d'une surface externe de la partie inférieure et d'une surface interne à la partie supérieure. De préférence, le pas de vis correspondant est optimisé de pour constituer un compromis entre le besoin d'étanchéité de ce couplage mécanique et celui de rendre la commutation entre les deux configurations de la partie supérieure à la fois rapide et pratique. Par exemple, le diamètre des hélicoïdes correspondantes est d'environ (à 10% près) 1,00 cm, et le nombre de tour est d'environ 1,8. Ainsi, le nombre de tours à effectuer pour changer la configuration est très petit, de sorte que cette opération peut être effectuée d'une main en évitant des troubles musculosquelettiques. De préférence, les hélicoïdes correspondant à ces filetages sont inclinées, de façon à renforcer l'étanchéité au niveau du couplage mécanique par vissage lorsque la partie supérieure est en configuration de fermeture.

De manière plus générale, le couplage mécanique entre la partie supérieure et la partie inférieure du bouchon est de préférence réalisé par un vissage entre une butée inférieure correspondant à la configuration de fermeture et une butée supérieure correspondant à la configuration d'ouverture. Ces butées contribuent au contrôle du couplage mécanique en empêchant tant de visser ou de dévisser de façon excessive la partie supérieure, ce qui pourrait détériorer le bouchon ou faire tomber la partie supérieure. La butée inférieure contribue aussi à l'étanchéité du bouchon lorsque la partie supérieure est en configuration de fermeture.

De préférence, la partie supérieure comprend une partie striée externe pour faciliter la préhension de celle-ci par une seule main, et rendre le changement de configuration de la partie supérieure par vissage ou dévissage pratique et aisée.

Dans le cadre de ces modes de réalisation, l'embout du bouchon est alors de préférence formé par la pointe de la partie inférieure et la portion supérieure de la partie supérieure, de telle sorte que, lorsque la partie supérieure est dans la configuration d'ouverture, le mélange puisse être distribué par le trou débouchant supérieur via un interstice entre la pointe et la portion supérieure, ce dernier étant en communication fluidique avec l'espace intérieur de la partie inférieure via la au moins une ouverture. Cette réalisation de l'embout intègre avantageusement les avantages des modes de réalisation précédant du bouchon, et permet notamment une distribution efficace, contrôlée et pratique du mélange dans tout type de tubes de test, sans formation de goutte excédentaire.

L'invention propose également une utilisation du dispositif selon l'invention pour traiter un échantillon de matière fécale. Le traitement est ainsi rendu facile et pratique, à l'image des avantages procurés par le dispositif selon l'invention.

Plus généralement, un autre objet de l'invention est de fournir une méthode de traitement d'un échantillon de matière fécale plus facile et pratique à mettre en œuvre. À cet effet, la présente invention propose une méthode selon la revendication 12.

L'utilisation du dispositif selon l'invention confère naturellement à la méthode de traitement selon l'invention son caractère pratique et aisé. En général, tous les modes de réalisation et avantages du dispositif selon l'invention se transposent *mutatis mutandis* à la présente méthode de traitement.

Dans le cadre du présent document, le verbe « retourner », sa conjugaison et le terme de « retournement » en référence au contenant, notamment en étape (iv) de la méthode susmentionnée, ne doivent pas être interprétés limitativement comme une rotation de 180° du dispositif autour d'un point central, mais plutôt de préférence comme une inversion de la relation de position en hauteur du bouchon et d'une base du contenant, à savoir que le contenant passe d'une position où le bouchon surmonte la base en une position ou la base surmonte le bouchon. Ainsi, l'étape (iv) correspond par exemple à retourner le contenant de façon ce qu'il ait une position oblique orientée « vers le bas » c'est-à-dire en dirigeant le bouchon « vers le bas », la base du contenant surmontant alors le reste du contenant.

La distribution de l'étape (v) se fait de préférence dans des tubes de test de petite taille, l'embout étant placé successivement au niveau d'ouvertures d'entrée de chaque tube. L'étape (v) comprenant de préférence d'appliquer d'une pression sur le contenant pour faciliter la distribution du mélange par l'embout.

### Brève description des figures

D'autres caractéristiques et avantages de la présente invention apparaîtront à la lecture de la description détaillée qui suit pour la compréhension de laquelle on se reportera aux figures annexées parmi lesquelles :

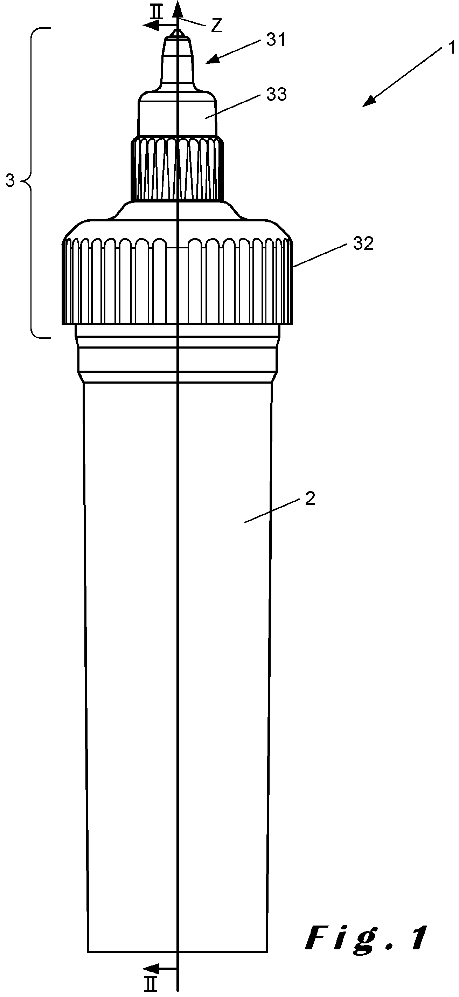

- la figure 1 illustre une vue extérieure du dispositif selon un mode de réalisation préféré de l'invention lorsqu'il est complètement assemblé, en particulier, dans le cas où le bouchon ferme le contenant ;
- la figure 2 illustre une vue en coupe du dispositif de la figure 1 selon le plan II ;

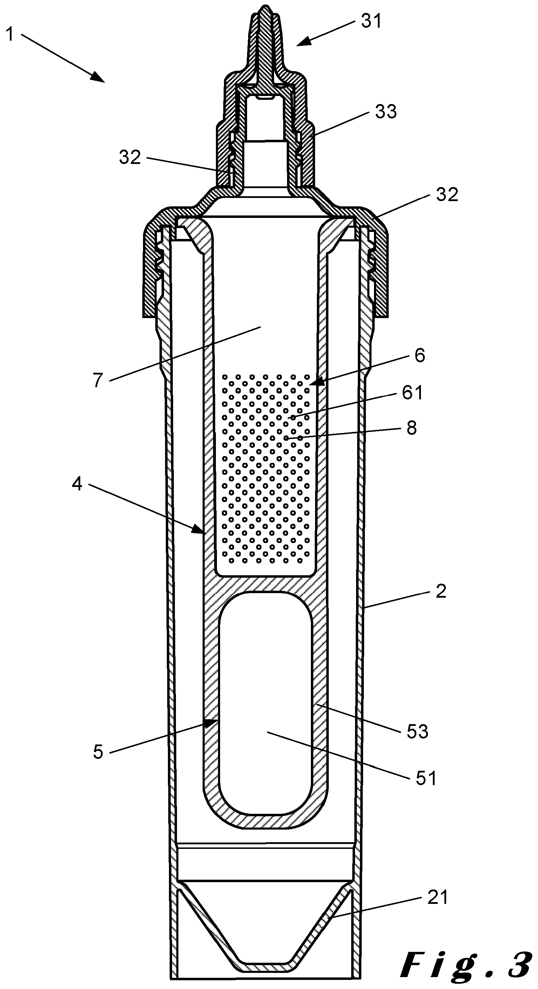

- la figure 3 illustre une vue en coupe du dispositif de la figure 1 selon le plan III ;
- la figure 4 illustre un agrandissement de la zone IV de la figure 2 ;

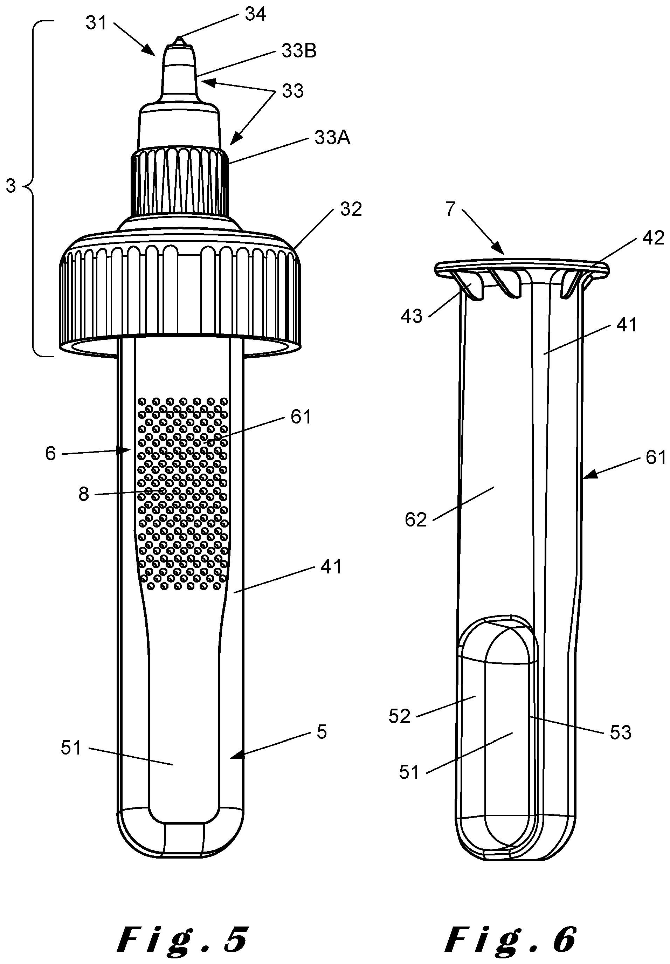

- la figure 5 illustre une vue globale tridimensionnelle arrière de la cuillère et du bouchon du dispositif de la figure 1 solidarisés et séparés du contenant ;
- la figure 6 illustre une vue globale tridimensionnelle à la fois avant et latérale de la cuillère de la figure 5 désolidarisée du bouchon ;

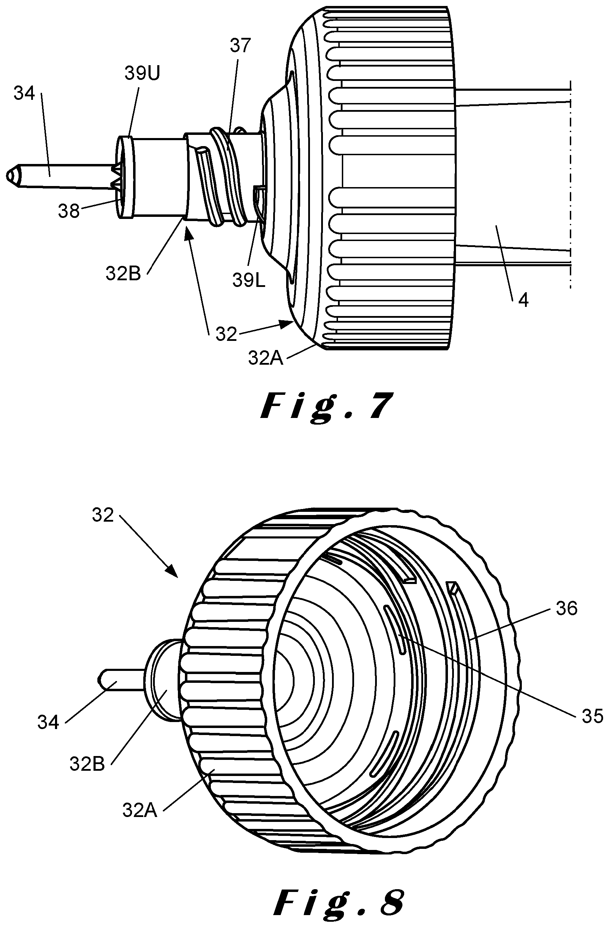

- la figure 7 illustre une vue tridimensionnelle avant de la cuillère et du bouchon de la figure 5 où la partie supérieure du bouchon a été enlevée, seule la partie inférieure du bouchon apparaissant ;
- la figure 8 illustre une vue globale tridimensionnelle partiellement intérieure de la partie inférieure du bouchon de la figure 7.

Les dessins des figures ne sont pas à l'échelle. Des éléments semblables sont en général dénotés par des références semblables dans les figures. Dans le cadre du présent document, les éléments identiques ou analogues peuvent porter les mêmes références. En outre, la présence de numéros ou lettres de référence aux dessins n'est aucunement limitative, y compris lorsque ces numéros ou lettres sont indiqués dans les revendications.

### Description détaillée d'un mode de réalisation de l'invention

Il est décrit ci-dessous en détails et principalement structurellement un mode de réalisation spécifique et préféré du dispositif selon l'invention. Cette description est faite en référence à des figures mais l'invention n'est pas limitée par celles-ci, ni par le mode de réalisation décrit. En particulier, les dessins ou figures décrits ci-dessous ne sont que schématiques et ne sont pas limitants.

Le dispositif 1 selon ce mode de réalisation particulier comprend un flacon constitué d'un contenant 2 et d'un bouchon 3. Le contenant est de forme tubulaire et s'étend autour d'un axe Z. Il est adapté pour renfermer une solution de mise en suspension d'un échantillon de matière fécale aux fins de son traitement. Il a une base jupée pour faciliter son maintien sans portoir.

Le contenant 2 comprend une seule ouverture supérieure apte à être fermée par le bouchon 3. Cette ouverture du contenant 2 surmonte le reste du contenant 2. Le bouchon 3 permet de fermer le contenant 2 autour de l'ouverture supérieure de façon amovible par vissage.

Le bouchon 3 a une structure double comprenant une partie supérieure 33 et d'une partie inférieure 32 surmontée par la partie supérieure 33 et qui peut être vissée sur une surface extérieure du contenant 2 autour de l'ouverture supérieure notamment via un filetage 36 sur une surface intérieure de la partie inférieure 32.

La partie inférieure 32 comprend une portion supérieure 32B et une portion inférieure 32A surmontée par la portion supérieure 32B, en une seule pièce. La portion inférieure 32A est dimensionnée pour s'adapter à l'ouverture supérieure par vissage, la surface intérieure comprenant le filetage 36 susdit étant celle de la portion inférieure 32A. La portion supérieure 32B est de plus petit diamètre et est muni sur une surface extérieure d'un autre filetage 37 agencé pour coopérer avec un filetage interne d'une portion inférieure 33A de la partie supérieure 33, de façon à coupler mécaniquement par vissage les parties supérieure 33 et inférieure 32. Ce couplage comprend de préférence deux butées 39L-U pour limiter le vissage vers le haut et vers le bas.

La portion supérieure 32B comprend une surface de sommet surmontant le reste de la portion supérieure 32B percée d'ouvertures 38, par exemple quatre, de petite dimension entourant symétriquement une pointe 34 qui surmonte le reste de la partie inférieure.

La partie supérieure 33 comprend, en plus de la portion inférieure 33A, une portion supérieure 33B de taille plus petite que la portion inférieure 33A, formant une seule pièce avec celle-ci et surmontant celle-ci.

La portion supérieure 33B forme une enveloppe pour la pointe 34 et inclut un trou débouchant supérieur et central qui peut être traversé hermétiquement par la pointe 34, lorsque la partie supérieure 33 est vissée sur la partie inférieure 32, de façon à ce que le bouchon 3 ferme totalement le contenant.

Lorsque la partie supérieure 33 est dévissée (vers le haut selon l'axe Z) de la partie inférieure 32, jusqu'à une des butées (supérieure 39U), la pointe 34 descend sous ce trou et un interstice entre la pointe 34 et la portion supérieure 33B permet un passage de fluide à partir des ouvertures 38 jusqu'au trou en question. Ainsi, selon que la partie supérieure 33 soit vissée contre une des butées (inférieure 39L) ou dévissée contre l'autre butée (supérieure 39U), elle est dans une configuration de fermeture ou d'ouverture, empêchant ou permettant ce passage.

La portion supérieure 33B forme ainsi avec la pointe 34 un embout 31 apte à distribuer un mélange de la solution de mise en suspension avec l'échantillon de matière fécale.

Des zones de renfort d'étanchéité peuvent en outre être prévues au niveau de liaisons entre différentes parties et portions du bouchon 3 et le contenant 2 tel que le comprendra l'homme du métier.

Les portions inférieures 32A et 33A ont une surface extérieure striées pour faciliter leur préhension et les opérations de vissage de dévissage.

Le dispositif 1 comprend une cuillère 4 pour prélever cet échantillon dont le manche 6 est solidarisé à ladite surface intérieure de la portion inférieure 32A. La solidarisation est réalisée de façon amovible, par coopération entre, d'une part, des languettes de maintien 35, par exemple cinq, sur la surface intérieure de la portion inférieure 32A, et d'autre part, un rebord 43 en une extrémité non opérante du manche 6 qui peut être insérée et maintenue par les languettes de maintien 35, cette solidarisation étant en outre améliorée par des renforts 43 au niveau de la partie inférieure 32.

La cuillère 4 comprend en son autre extrémité une partie opérante 5 creuse calibrée pour contenir l'échantillon. Un espace creux de la partie opérante 5 apte à contenir cet échantillon est défini par un fond 51 et un bord latéral 52 entourant le fond 51. Le manche 6 s'étend de la partie opérante 5 à la surface intérieure de la portion inférieure 32A.

L'espace creux est d'une forme principalement parallélépipédique rectangle aux angles et/ou coins arrondis, pour améliorer l'homogénéisation du mélange de l'échantillon avec la solution de mise en suspension.

Lorsque le bouchon 3, ou plus précisément la partie inférieure 32, ferme le contenant 2, quelle que soit la configuration en termes de (dé)vissage de la partie supérieure 33 entre les deux butées 39L-U, la cuillère 4 s'étend dans le contenant 2 le long de l'axe Z, à proximité, par exemple 2,5 cm selon les mesures des modes de réalisation fournies dans l'exposé, de la base 21.

Le manche 6 et la partie opérante 5 sont formées à partir d'une même forme parallélépipédique rectangle dans laquelle est formée l'espace ceux susdit. Un bord en biseau 41 de la cuillère 4 peut être prévu pour faciliter l'homogénéisation du mélange, de sorte que cette forme possède des angles légèrement arrondis ou non abruptes.

Le manche 6 est creux, et comprend une cavité interne 7 en communication fluidique avec l'intérieur de la partie inférieure 32 du bouchon 3, donc aussi avec l'embout 31 via les ouvertures 38.

Le manche 6 consiste essentiellement en quatre parois planes formant le parallélépipède susdit (et marginalement en des jonctions courbes entre elles), dont une paroi avant 62 qui, si elle se prolongeait, renfermerait l'espace creux, ou en d'autres termes, à partir de laquelle est formé cet espace creux, et une paroi arrière 61, opposée à la paroi avant 62, et prolongeant continument régulièrement selon le même plan le fond 51 de la partie opérante 5.

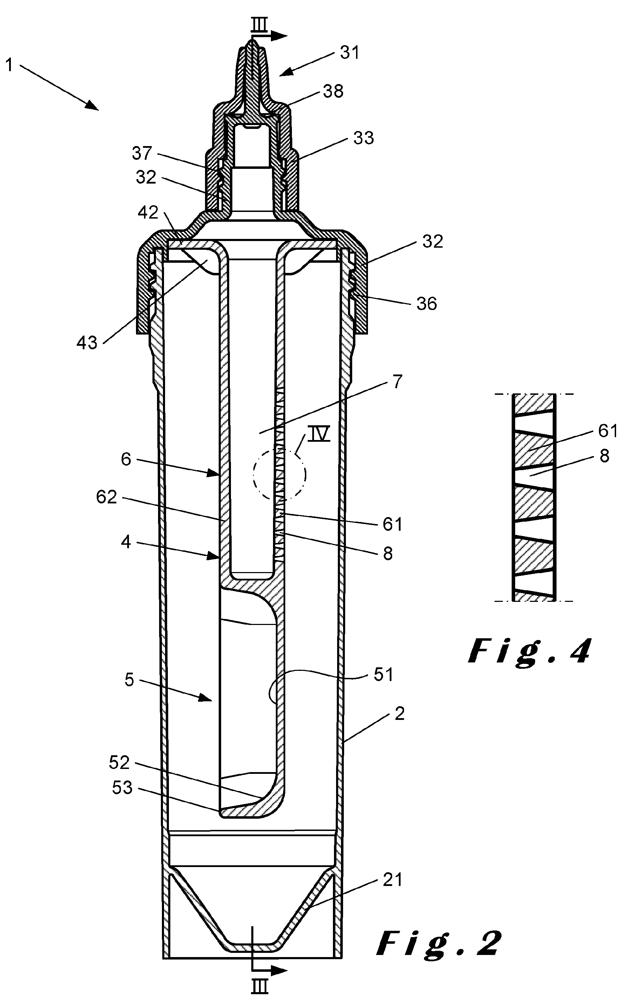

Des orifices de filtration 8 sont prévus uniquement sur la paroi arrière 61. Ils permettent une communication fluidique l'espace intérieur du contenant 2 et cette cavité interne 7, donc avec l'embout 31. Les orifices de filtration 8 jouissent d'une position qui les protègent d'une obstruction par de la matière fécale lors de l'étape de prélèvement de l'échantillon. Comme il est visible en figure 4, les orifices sont de forme tronconique orientée vers du contenant 2 vers la cavité interne 7.

La paroi avant 62 et les deux parois latérales du manche 6 qui la relient à la paroi arrière 61 sont pleines. Ceci est avantageux dans le cas de la paroi avant 62 pour racler de la matière fécale excédentaire lors du prélèvement sur le bord du contenant 2, vers l'extérieur de celui-ci, de façon continue sur le sommet 53 du bord latéral 52 et sur la paroi avant 62, ce qui permet une meilleure calibration du prélèvement et donc une meilleure reproductibilité du traitement.

En résumé, l'invention concerne un dispositif 1 pour traiter un échantillon de matière fécale comprenant un contenant 2 qui peut être fermé par un bouchon 3 auquel peut être solidarisé un manche 6 d'une cuillère 4 apte à s'étendre dans le contenant 2. Le manche 6 inclut une cavité 7 en communication fluidique avec le bouchon 3 lorsqu'ils sont solidarisés ensemble, et des orifices 8 de filtration sur une paroi du manche 6, préférentiellement une paroi arrière 61 opposée à une paroi avant 62 du manche 6 à partir de laquelle est formée une partie opérante 5 creuse de la cuillère 4.

Dans le cadre de ce document, le terme « surmonter » et ses conjugaisons sont de préférence à considérer selon l'axe Z. Un élément en surmonte un autre s'il suit celui-ci selon l'axe Z. L'axe Z est notamment dirigé « du bas vers le haut ».

La présente invention a été décrite dans cette section au regard d'un mode de réalisation spécifique qui a une valeur purement illustrative et ne doit pas être considéré comme limitatif. Il apparaîtra directement pour l'homme du métier que l'invention n'est pas limitée aux exemples illustrés ou décrits ci-dessus, et que sa portée est plus largement définie par les revendications ci-après introduites.

## Revendications

1. Dispositif (1) pour traiter un échantillon de matière fécale, comprenant :
- un contenant (2) pour contenir une solution de traitement de l'échantillon ;
- un bouchon (3) pour fermer le contenant (2) de façon amovible, le bouchon (3) comprenant un embout (31) pour distribuer un mélange de la solution avec l'échantillon ;
- une cuillère (4) pour prélever l'échantillon comprenant :
• une partie opérante (5) creuse comprenant un fond (51) et un bord latéral (52) entourant le fond (51), et
• un manche (6) s'étendant à partir de la partie opérante (5),
le manche (6) étant solidarisé avec le bouchon (3),
la cuillère (4) étant dimensionnée pour s'étendre dans le contenant (2) lorsque le bouchon (3) ferme celui-ci ;
dans lequel le manche (6) comprend :
- une cavité interne (7) en communication fluidique avec l'embout (31) du bouchon (3) ;
- une paroi arrière (61) prolongeant le fond (51) de la partie opérante (5), bordant la cavité interne (7) et comprenant des orifices de filtration (8) du mélange ;
dans lequel le manche (6) comprend en outre une paroi avant (62) opposée à la paroi arrière (61) et bordant la cavité interne (7). et dans lequel les parois avant (62) et arrière (61) sont planes et parallèles;
**caractérisé en ce que**
la paroi avant est pleine.

2. Dispositif (1) selon la revendication 1, dans lequel le contenant (2) s'étend autour d'un axe (Z), et dans lequel :
- au moins 15% en volume du contenant (2) s'étend au-dessus des orifices de filtration (8) le long de l'axe (Z), et
- au moins 15% en volume du contenant (2) s'étend en-dessous des orifices de filtration (8) le long de l'axe (Z),
lorsque le bouchon (3) ferme le contenant (2).

3. Dispositif (1) selon la revendication 2, dans lequel le contenant (2) est de forme tubulaire ayant une hauteur mesurée le long de l'axe (Z) et un diamètre de section transverse mesuré perpendiculairement à l'axe (Z) au moins trois fois plus petit, de préférence quatre fois plus petit, que cette hauteur, la cuillère (4) étant dimensionnée pour s'étendre dans le contenant (2), à partir du bouchon (3), le long de l'axe (Z), sur au moins 70% de cette hauteur.

4. Dispositif (1) selon l'une quelconque des revendications précédentes, dans lequel le fond (51) et le bord latéral (52) de la partie opérante (5) définissent un espace creux de forme parallélépipédique rectangle de coins et/ou angles de préférence arrondis.

5. Dispositif (1) selon la revendication 4, dans lequel le manche (6) prolonge continument la partie opérante (5) selon une même forme prismique de coins et/ou d'angles de préférence arrondis dans laquelle est formé l'espace creux.

6. Dispositif (1) selon l'une quelconque des revendications précédentes, dans lequel tout ou partie des orifices de filtration (8) définissent chacun une forme tronconique dans la paroi arrière (61) de diamètre de section compris entre 0,10 mm et 1,00 mm et décroissant du contenant (2) vers la cavité interne (7).

7. Dispositif (1) selon l'une quelconque des revendications précédentes, dans lequel le bouchon (3) comprend :
- une partie inférieure (32) munie d'éléments de fixation amovible (36) au contenant (2), autour d'une ouverture supérieure de celui-ci, et d'au moins une ouverture (38) permettant une communication fluidique entre l'embout (31) du bouchon (3) et un espace intérieur de la partie inférieure (32) du bouchon (3) prolongeant continument la cavité intérieure (7) ;
- une partie supérieure (33) surmontant la partie inférieure (32) et couplée mécaniquement à celle-ci, de sorte qu'elle puisse être déplacée par rapport à celle-ci entre :
• une configuration de fermeture empêchant une distribution du mélange par l'embout (31), et
• une configuration d'ouverture permettant cette distribution.

8. Dispositif (1) selon la revendication 7, dans lequel :
- la partie inférieure (32) comprend en outre une pointe (34) surmontant la au moins une ouverture (38) ;
- la partie supérieure (33) comprend une portion supérieure (33B) dimensionnée pour envelopper au moins partiellement la pointe (34) et percée par un trou débouchant supérieur dimensionné pour accueillir la pointe (34) de façon à être hermétiquement obturé lorsque la partie supérieure (33) est dans la configuration de fermeture.

9. Dispositif (1) selon la revendication 8, dans lequel l'embout (31) est formé par la pointe (34) de la partie inférieure (32) et la portion supérieure (33B) de la partie supérieure (33), de telle sorte que, lorsque la partie supérieure (33) est dans la configuration d'ouverture, le mélange puisse être distribué par le trou débouchant supérieur via un interstice entre la pointe (34) et la portion supérieure (33B) qui est en communication fluidique avec l'espace intérieur de la partie inférieure (32) via la au moins une ouverture (38).

10. Dispositif (1) selon l'une quelconque des revendications 7 à 9, dans lequel le couplage mécanique entre la partie supérieure (33) et la partie inférieure (32) du bouchon (3) est réalisé par un vissage entre une butée inférieure (39L) correspondant à la configuration de fermeture et une butée supérieure (39U) correspondant à la configuration d'ouverture.

11. Dispositif (1) selon l'une quelconque des revendications précédentes, dans lequel le contenant (2) est constitué de polyéthylène basse densité linéaire.

12. Méthode de traitement d'un échantillon de matière fécale au moyen d'un dispositif (1) selon l'une quelconque des revendications précédentes, comprenant les étapes suivantes, dans cet ordre :
(i) prélever l'échantillon au moyen de la cuillère (4), de sorte que l'échantillon se trouve dans la partie opérante (5) de la cuillère (4) ;
(ii) placer la cuillère (4) avec l'échantillon dans le contenant (2) préalablement rempli de solution de traitement, et fermer le contenant (2) au moyen du bouchon (3) ;
(iii) agiter mécaniquement le contenant (2) pour mélanger la solution de traitement avec l'échantillon ;
(iv) retourner le contenant (2) de façon à ce que du mélange de la solution de traitement avec l'échantillon obtenu à l'étape (iii) soit filtré à travers les orifices de filtration (8), et circule dans la cavité interne (7) du manche (6) jusqu'à l'embout (31) du bouchon (3) ;
(v) distribuer de ce mélange au moyen de l'embout (31) ;
dans laquelle l'étape (i) comprend un raclage continu de matière fécale sur la paroi avant (61) du manche (6) et sur le dessus de la partie opérante (5) de la cuillère (4).

13. Méthode selon la revendication 12, dans laquelle la distribution de l'étape (v) se fait dans des tubes de test et comprend un placement de l'embout (31) successivement au niveau d'ouvertures d'entrée des tubes de test.

## Patentansprüche

1. Vorrichtung (1) zum Behandeln einer Probe aus Fäkalmaterial, umfassend:
- einen Behälter (2) zum Enthalten einer Lösung zur Behandlung der Probe;
- einen Verschluss (3) zum Verschließen des Behälters (2) auf abnehmbare Weise, wobei der Verschluss (3) eine Tülle (31) zum Abgeben einer Mischung der Lösung mit der Probe umfasst;
- einen Löffel (4) zum Entnehmen der Probe, umfassend:
- ein hohles, operatives Teil (5), das einen Boden (51) und einen seitlichen Rand (52) umfasst, der den Boden (51) umgibt, und
- einen Griff (6), der sich ausgehend von dem operativen Teil (5) erstreckt, wobei der Griff (6) mit dem Verschluss (3) fest verbunden ist, wobei der Löffel (4) bemessen ist, um sich in den Behälter (2) zu erstrecken, wenn der Verschluss (3) diesen verschließt;
wobei der Griff (6) umfasst:
- einen internen Hohlraum (7) in strömungstechnischer Kommunikation mit der Tülle (31) des Verschlusses (3);
- eine hintere Wand (61), die den Boden (51) des operativen Teils (5) verlängert, angrenzend an den internen Hohlraum (7) und Bohrungen (8) zur Filtration der Mischung umfassend;
wobei der Griff (6) weiter eine vordere Wand (62), der hinteren Wand (61) gegenüberliegend und an den internen Hohlraum (7) angrenzend, umfasst,
und wobei die vordere (62) und hintere (61) Wand flach und parallel sind;
**dadurch gekennzeichnet, dass**
die vordere Wand massiv ist.

2. Vorrichtung (1) nach Anspruch 1, wobei der Behälter (2) sich um eine Achse (Z) herum erstreckt und wobei:
- mindestens 15 Volumen-% des Behälters (2) sich entlang der Achse (Z) über den Bohrungen (8) zur Filtration erstreckt, und
- mindestens 15 Volumen-% des Behälters (2) sich entlang der Achse (Z) unter den Bohrungen (8) zur Filtration erstreckt, wenn der Verschluss (3) den Behälter (2) verschließt.

3. Vorrichtung (1) nach Anspruch 2, wobei der Behälter (2) von röhrenförmiger Form ist und eine Höhe, gemessen entlang der Achse (Z), und einen Durchmesser im Querschnitt, gemessen senkrecht zu der Achse (Z), mindestens dreimal kleiner, vorzugsweise viermal kleiner als diese Höhe aufweist, wobei der Löffel (4) bemessen ist, um sich ausgehend von dem Verschluss (3) entlang der Achse (Z) über mindestens 70 % dieser Höhe in den Behälter (2) zu erstrecken.

4. Vorrichtung (1) nach einem der vorstehenden Ansprüche, wobei der Boden (51) und der seitliche Rand (52) des operativen Teils (5) einen hohlen Raum von parallelepipedischer Rechteckform mit vorzugsweise abgerundeten Ecken und/oder Winkeln definieren.

5. Vorrichtung (1) nach Anspruch 4, wobei der Griff (6) das operative Teil (5) gemäß einer gleichen prismatischen Form mit vorzugsweise abgerundeten Ecken und/oder Winkeln kontinuierlich verlängert, in der der hohle Raum gebildet ist.

6. Vorrichtung (1) nach einem der vorstehenden Ansprüche, wobei alle oder ein Teil der Bohrungen (8) zur Filtration in der hinteren Wand (61) jeweils eine kegelstumpfförmige Form mit einem Schnittdurchmesser zwischen 0,10 mm und 1,00 mm und von dem Behälter (2) in Richtung des internen Hohlraums (7) abnehmend definieren.

7. Vorrichtung (1) nach einem der vorstehenden Ansprüche, wobei der Verschluss (3) umfasst:
- ein unteres Teil (32), versehen mit Elementen (36) zur abnehmbaren Befestigung an dem Behälter (2) um eine obere Öffnung von diesem herum, und mindestens eine Öffnung (38), die eine strömungstechnische Kommunikation zwischen der Tülle (31) des Verschlusses (3) und einem internen Raum des unteren Teils (32) des Verschlusses (3), der den internen Hohlraum (7) kontinuierlich verlängert, erlaubt;
- ein oberes Teil (33), das das untere Teil (32) überbrückt und mechanisch derart mit diesem gekoppelt ist, dass es bezüglich diesem verschoben werden kann zwischen:
- einer Verschlusskonfiguration, die eine Abgabe der Mischung durch die Tülle (31) verhindert, und
- einer Öffnungskonfiguration, die diese Abgabe erlaubt.

8. Vorrichtung (1) nach Anspruch 7, wobei:
- das untere Teil (32) weiter eine Spitze (34) umfasst, die die mindestens eine Öffnung (38) überbrückt;
- das obere Teil (33) einen oberen Abschnitt (33B) umfasst, bemessen zum mindestens teilweisen Umhüllen der Spitze (34) und durch ein oberhalb mündendes Loch durchstochen, bemessen zur Aufnahme der Spitze (34) auf eine Weise, um hermetisch verstopft zu werden, wenn das obere Teil (33) in der Verschlusskonfiguration vorliegt.

9. Vorrichtung (1) nach Anspruch 8, wobei die Tülle (31) durch die Spitze (34) des unteren Teils (32) und den oberen Abschnitt (33B) des oberen Teils (33) derart gebildet wird, dass, wenn das obere Teil (33) in der Öffnungskonfiguration vorliegt, die Mischung durch das oberhalb mündende Loch über einen Zwischenraum zwischen der Spitze (34) und dem oberen Abschnitt (33B) abgegeben werden kann, der über die mindestens eine Öffnung (38) in strömungstechnischer Kommunikation mit dem internen Raum des unteren Teils (32) vorliegt.

10. Vorrichtung (1) nach einem der Ansprüche 7 bis 9, wobei die mechanische Kopplung zwischen dem oberen Teil (33) und dem unteren Teil (32) des Verschlusses (3) durch eine Verschraubung zwischen einem unteren Anschlag (39L), die der Verschlusskonfiguration entspricht, und einem oberen Anschlag (39U), die der Öffnungskonfiguration entspricht, hergestellt wird.

11. Vorrichtung (1) nach einem der vorstehenden Ansprüche, wobei der Behälter (2) aus linearem Polyethylen niederer Dichte besteht.

12. Verfahren zur Behandlung einer Probe aus Fäkalmaterial mittels einer Vorrichtung (1) nach einem der vorstehenden Ansprüche, umfassend die folgenden Schritte in dieser Reihenfolge:
(i) Entnehmen der Probe mittels des Löffels (4) derart, dass die Probe sich in dem operativen Teil (5) des Löffels (4) befindet;
(ii) Platzieren des Löffels (4) mit der Probe in den Behälter (2), der zuvor mit Behandlungslösung gefüllt wurde, und Schließen des Behälters (2) mittels des Verschlusses (3);
(iii) mechanisches Schütteln des Behälters (2) zum Mischen der Behandlungslösung mit der Probe;
(iv) Umdrehen des Behälters (2) auf eine Weise, dass die Mischung der Behandlungslösung mit der Probe, die in Schritt (iii) erhalten wurde, durch die Bohrungen (8) zur Filtration hindurch gefiltert wird und in den internen Hohlraum (7) des Griffs (6) bis zu der Tülle (31) des Verschlusses (3) fließt;
(v) Abgeben dieser Mischung mittels der Tülle (31);
wobei der Schritt (i) ein kontinuierliches Abstreifen des Fäkalmaterials auf der vorderen Wand (61) des Griffs (6) und auf der Oberseite des operativen Teils (5) des Löffels (4) umfasst.

13. Verfahren nach Anspruch 12, wobei die Abgabe von Schritt (v) in Teströhrchen erfolgt und eine Platzierung der Tülle (31) nacheinander auf Höhe der Einlassöffnungen der Teströhrchen umfasst.

## Claims

1. A device (1) for treating a sample of fecal matter, comprising:
- a container (2) for containing a solution for treating the sample;
- a plug (3) for removably closing the container (2),
the plug (3) comprising an end piece (31) for distributing a mixture of the solution with the sample;
- a spoon (4) for collecting the sample comprising:
• a hollow operating portion (5) comprising a bottom (51) and a lateral edge (52) surrounding the bottom (51), and
• a handle (6) extending from the operating portion (5),
the handle (6) being secured to the plug (3),
the spoon (4) being dimensioned to extend into the container (2) when the plug (3) closes the latter;
wherein the handle (6) comprises:
- an internal cavity (7) in fluid communication with the end piece (31) of the plug (3);
- a rear wall (61) extending the bottom (51) of the operating portion (5), bordering the internal cavity (7) and comprising orifices (8) for filtering the mixture;
wherein the handle (6) also comprises a front wall (62) opposite the rear wall (61) and bordering the internal cavity (7), and wherein the front (62) and rear (61) walls are flat and parallel;
**characterized in that** the front wall is solid.

2. The device (1) according to claim 1, wherein the container (2) extends around an axis (Z), and wherein:
- at least 15% by volume of the container (2) extends above the filtration orifices (8) along the axis (Z), and
- at least 15% by volume of the container (2) extends below the filtration orifices (8) along the axis (Z),
when the plug (3) closes the container (2).

3. The device (1) as claimed in claim 2, wherein the container (2) is tubular in shape having a height measured along the axis (Z) and a cross-sectional diameter measured perpendicular to the axis (Z) at least three times smaller, preferably four times smaller, than that height, the spoon (4) being dimensioned to extend into the container (2) from the plug (3) along the axis (Z) over at least 70% of that height.

4. The device (1) according to any one of the preceding claims, wherein the bottom (51) and the lateral edge (52) of the operating portion (5) define a hollow space of rectangular parallelepiped shape with preferably rounded corners and/or angles.

5. The device (1) according to claim 4, wherein the handle (6) is a continuous extension of the operating portion (5) in the same prismatic shape of preferably rounded corners and/or angles wherein the hollow space is formed.

6. The device (1) according to any one of the preceding claims, wherein all or some of the filtration orifices (8) each define a frustoconical shape in the rear wall (61) with a cross-sectional diameter of between 0.10 mm and 1.00 mm and decreasing from the container (2) towards the internal cavity (7).

7. The device (1) according to any one of the preceding claims, wherein the plug (3) comprises:
- a lower portion (32) equipped with elements (36) for removably attaching to the container (2), around an upper opening thereof, and at least one opening (38) allowing a fluid communication between the end piece (31) of the plug (3) and an interior space of the lower portion (32) of the plug (3) continuously extending the interior cavity (7);
- an upper portion (33) surmounting the lower portion (32) and mechanically coupled thereto, so that it may be moved relative thereto between:
• a closed configuration preventing a distribution of the mixture through the end piece (31), and
• an opening configuration allowing this distribution.

8. The device (1) according to claim 7, wherein:
- the lower portion (32) also comprises a tip (34) surmounting the at least one opening (38),
- the upper portion (33) comprises an upper segment (33B) dimensioned to at least partially enclose the tip (34) and pierced by an upper through hole dimensioned to receive the tip (34) so as to be hermetically clogged when the upper portion (33) is in the closed configuration.

9. The device (1) of claim 8, wherein the end piece (31) is formed by the tip (34) of the lower portion (32) and the upper segment (33B) of the upper portion (33), such that, when the upper portion (33) is in the open configuration, the mixture may be distributed through the upper through-hole via an interstice between the tip (34) and the upper segment (33B) which is in fluid communication with the interior space of the lower portion (32) via the at least one opening (38).

10. The device (1) according to any one of claims 7 to 9, wherein the mechanical coupling between the upper portion (33) and the lower portion (32) of the plug (3) is produced by a screw connection between a lower stop (39L) corresponding to the closed configuration and an upper stop (39U) corresponding to the open configuration.

11. The device (1) according to any one of the preceding claims, wherein the container (2) is made of linear low density polyethylene.

12. A method for treating a sample of fecal matter by means of a device (1) according to any one of the preceding claims, comprising the following steps, in this order:
(i) collecting the sample using the spoon (4), so that the sample is in the operating portion (5) of the spoon (4);
(ii) placing the spoon (4) with the sample in the container (2) previously filled with treatment solution, and close the container (2) by means of the plug (3);
(iii) mechanically stirring the container (2) to mix the treatment solution with the sample;
(iv) turning the container (2) upside down so that the mixture of the treatment solution with the sample obtained in step (iii) is filtered through the filtration orifices (8) and circulates in the internal cavity (7) of the handle (6) as far as the end piece (31) of the plug (3);
(v) distributing this mixture through the end piece (31);
wherein step (i) comprises a continuous scraping of fecal matter from the front wall (61) of the handle (6) and from the top of the operating portion (5) of the spoon (4).

13. The method of claim 12, wherein the distributing of step (v) is into test tubes and comprises placing the end piece (31) successively at the level of the inlet openings of the test tubes.
